# EUROPÄISCHE PATENTSCHRIFT

(11) **EP 3 979 953 B1**
(45) Veröffentlichungstag und Bekanntmachung des Hinweises auf die Patenterteilung: **29.07.2026**
(21) Anmeldenummer: 20731445.1
(22) Anmeldetag: 05.06.2020
(51) Int. Cl.: A61F 2/68, A61B 5/11, A61F 2/70

(54) **VERFAHREN ZUM BETREIBEN EINER ORTHOPÄDIETECHNISCHEN EINRICHTUNG UND ENTSPRECHENDE EINRICHTUNG**
METHOD FOR OPERATING AN ORTHOPEDIC DEVICE AND CORRESPONDING ORTHOPEDIC DEVICE
PROCÉDÉ POUR FAIRE FONCTIONNER UN DISPOSITIF ORTHOPÉDIQUE ET DISPOSITIF CORRESPONDANT

(30) Priorität: 05.06.2019 DE 102019115096
(43) Veröffentlichungstag der Anmeldung: 13.04.2022
(73) Patentinhaber: Otto Bock Healthcare Products GmbH, 1110 Wien (AT)
(72) Erfinder: SEIFERT, Dirk, 1070 Wien (AT); TSCHIEDEL, Michael, 1110 Wien (AT); HOFFMANN, Robert, 1160 Wien (AT)
(74) Vertreter: Gramm, Lins & Partner Patent- und Rechtsanwälte PartGmbB
(86) Internationale Anmeldenummer: PCT/EP2020/065690
(87) Internationale Veröffentlichungsnummer: WO 2020/245400

(56) Entgegenhaltungen:
- EP-A1- 2 616 115
- EP-A1- 2 863 846
- EP-A1- 3 156 010
- DE-B3- 102016 114 075
- JP-A- 2012 135 486
- JP-A- 2014 027 978

## Beschreibung

Die Erfindung betrifft ein Verfahren zum Betreiben einer orthopädietechnischen Einrichtung, die ein erstes Körperteil eines Trägers unterstützt oder ersetzt und wenigstens einen steuerbaren Aktuator aufweist, wobei das Verfahren die folgenden Schritte aufweist:
a) Bestimmen eines zeitlichen Verlaufs wenigstens eines Parameters, der eine Aussage über einen Bewegungszustand des Trägers erlaubt, aus Messwerten wenigstens eines Sensors,
b) Erkennen des Bewegungszustandes aus dem wenigstens einen bestimmten zeitlichen Verlauf und
c) Steuern des wenigstens einen steuerbaren Aktuators in Abhängigkeit des erkannten Bewegungszustandes,
wobei zum Erkennen des Bewegungszustandes zumindest auch der zeitliche Verlauf wenigstens eines Parameters eines zweiten Körperteiles des Trägers verwendet wird.

Derartige Verfahren sind beispielsweise aus der JP 2014 027978 A und der EP 3 156 010 A1 bekannt. Andere Verfahren zum Betreiben einer derartigen orthopädietechnischen Einrichtung sind der JP 2012 135486 A, der EP 2 863 846 A1 und der DE 10 2016 114075 B3 aus dem Hause der Anmelderin zu entnehmen.

Orthopädietechnische Einrichtungen sind insbesondere Prothesen und Orthesen, die für Extremitäten, also Arme und/oder Beine, eines Trägers hergestellt werden. Eine orthopädietechnische Einrichtung wird dabei in der Regel an einer einzigen Extremität, also einem einzigen Bein oder einem einzigen Arm, des Trägers angeordnet. Handelt es sich bei der orthopädietechnischen Einrichtung um eine Prothese, ersetzt sie wenigstens ein Körperteil. Dies kann beispielsweise ein Fuß, ein Knöchel oder ein Knie, aber auch ein Unterarm oder eine Hand sein. Es gibt selbstverständlich auch Prothesen, die mehrere Körperteile ersetzen. So ist beispielsweise eine Prothese, die für einen Oberschenkelamputierten hergestellt wird, eingerichtet, das Knie, den Knöchel und der Fuß zu ersetzen.

Eine Orthese hingegen unterstützt das jeweilige Körperteil. Dies umfasst einerseits das Stützen und Schützen vor Überbeanspruchung, beispielsweise in einem postoperativen Heilungsprozess, indem beispielsweise ein Winkelbereich, in dem ein Gelenk eingesetzt werden soll, durch eine Orthese begrenzt ist. Zum Unterstützen im Rahmen der vorliegenden Erfindung gehört auch das Stützen durch Entlasten, das beispielsweise bei Sport-Orthesen oder auch bei sogenannten Exoskeletten, also tragbaren und bei Bedarf mit Aktuatoren ausgestattete mechanische Strukturen, die im medizinischen Bereich beispielsweise in der Rehabilitation oder als Alternative zum Rollstuhl eingesetzt werden, erreicht wird. Es muss nicht zwangsweise eine Einschränkung des Anwenders vorliegen. Es ist beispielsweise auch mögliche eine solche Orthese / ein solches Exoskelett zu verwenden, um die Belastung des Körpers bei physischen Tätigkeiten zu reduzierten, die Leistungsfähigkeit zu steigern und/oder das Risiko einer Verletzung zu reduzieren.

Insbesondere wenn es sich bei dem ersten Körperteil um ein Bein oder den Amputationsstumpf eines Beines handelt, ist es wichtig und von großem Vorteil, den Bewegungszustand des Trägers zu kennen, um den steuerbaren Aktuator entsprechend anzupassen. Erkennbare Bewegungszustände sind beispielsweise aufwärts und abwärts Treppensteigen, das Gehen auf einer Rampe, das Gehen und Laufen in unterschiedlichen Geschwindigkeiten, Stehen, Sitzen, das Übersteigen von Hindernissen, die sich im Weg befinden oder auch eine für eine Routinearbeit charakteristische Bewegung. All diese unterschiedlichen Bewegungszustände benötigen oftmals unterschiedliche Steuerungen des steuerbaren Aktuators.

Wichtig ist dabei, zwischen einem Bewegungszustand des Trägers einer orthopädietechnischen Einrichtung, der oben beschrieben ist, und dem Bewegungszustand oder der Bewegung des jeweiligen Körperteils zu unterscheiden. Während des Gehens in der Ebene beispielsweise ändert sich der Bewegungszustand des Trägers der orthopädietechnischen Einrichtung nicht, so lange das Gehen in der Ebene andauert. Die Bewegung des Körperteiles, beispielsweise eines Knies hingegen ändert sich mehrfach in jedem Schritt. Es durchläuft Standphasen und Schwungphasen mit unterschiedlichen Schlüsselereignissen, wie beispielsweise dem Fersenauftritt. Auch das Erkennen und Vorhersagen dieser Schlüsselereignisse ist für die Steuerung einer orthopädietechnischen Einrichtung wichtig und aus dem Stand der Technik seit langem bekannt. Im Rahmen der hier beschriebenen Erfindung geht es in erster Linie jedoch um das Erkennen des Bewegungszustandes des Trägers der orthopädietechnischen Einrichtung und die Frage, wie ein Wechsel des Bewegungszustandes erkannt und die Steuerung der orthopädietechnischen Einrichtung entsprechend angepasst werden kann.

Der Bewegungszustand eines Trägers dauert folglich in der Regel mehrere Schrittzyklen lang an, während sich der Bewegungszustand eines Körperteils auf einer deutlich kürzeren Zeitskala ändert. Diese Änderung kann mehrfach innerhalb eines einzigen Schrittzyklus erfolgen. Der wenigstens eine steuerbare Aktuator ist vorzugsweise vorgesehen und eingerichtet, den Bewegungszustand eines Körperteils, nämlich des ersten Körperteils, das durch die orthopädietechnische Einrichtung unterstützt oder ersetzt wird, zu verändern. Er ist in der Regel nicht eingerichtet, um den Bewegungszustand des Trägers der orthopädietechnischen Einrichtung zu verändern.

Bei einem steuerbaren Aktuator kann es sich beispielsweise um ein Dämpfungselement, beispielsweise einen hydraulischen Dämpfer, handeln. Bei hydraulischen Dämpfern sind insbesondere Ventile in einer Fluidverbindung vorhanden, die vorzugsweise stufenlos geöffnet oder geschlossen werden können. Dadurch wird der Querschnitt der Fluidverbindung vergrößert oder verkleinert, wodurch der einem Fluidfluss entgegengesetzte Widerstand und damit die Dämpfung, die von dem Dämpfungselement hervorgerufen wird, verringert oder erhöht werden kann.

Bei dem steuerbaren Aktuator kann es sich auch um ein Stellglied handeln, durch das eine bestimmte Bewegung zumindest eines Teils der orthopädietechnischen Einrichtung oder der ganzen orthopädietechnischen Einrichtung gesteuert werden kann.

Dies ist beispielsweise bei aktiven orthopädietechnischen Einrichtungen, beispielsweise aktiven Kniegelenken oder aktiven Knöchelgelenken nötig, damit das Gelenk der orthopädietechnischen Einrichtung die gewünschte Funktion erfüllt. Steuerbare Aktuatoren können aktiv oder passiv ausgebildet sein, unabhängig davon, ob es sich um Dämpfungselemente oder Stellglieder handelt.

Als steuerbare Aktuatoren im Sinne der vorliegenden Erfindung werden auch Mittel und Methoden zur Stimulation des Bewegungsapparats angesehen, insbesondere der Elektrostimulation von Muskeln und Nerven, beispielsweise durch Elektroden. Diese können beispielsweise auf der Haut des Trägers angeordnet sein und durch elektrische Impulse unter der Haut liegende Muskeln stimulieren. Ebenso kann es sich um subkutane Elektroden handeln, beispielsweise am Nerv anliegende Elektroden.

Aus dem Stand der Technik ist seit langem bekannt, den steuerbaren Aktuator in Abhängigkeit des erkannten Bewegungszustandes zu steuern. Dazu ist der wenigstens eine Sensor eingerichtet, Messwerte aufzunehmen, aus denen wenigstens ein Parameter bestimmbar ist, dessen zeitlicher Verlauf Aussagen über einen Bewegungszustand erlaubt. Dabei muss der zeitliche Verlauf nicht über einen ganzen Schrittzyklus hinweg detektiert oder ausgewertet und dokumentiert werden. Der Parameter kann beispielsweise der Kniewinkel einer Knieprothese oder Knieorthese sein, der beispielsweise über einen Schrittzyklus hinweg gemessen und in der elektrischen Steuerung ausgewertet wird. Der dabei maximal auftretende Kniewinkel unterscheidet sich je nach Bewegungszustand. Der maximale Flexionswinkel, der beispielsweise bei einem Knie auftritt, ist deutlich größer, wenn der Träger der orthopädietechnischen Einrichtung eine Treppe nach oben steigt, als wenn er in der Ebene geht. Daraus kann auf den Bewegungszustand geschlossen werden, wobei diese Information dann verwendet wird, um den steuerbaren Aktuator beispielsweise in der Schwungphase des Beins so zu steuern, dass das Knie die gewünschte Bewegung ausführt.

An andere Stelle ist es beispielsweise sinnvoll, beim Treppensteigen in der Schwungphase der ipsilateralen Extremität den Fuß in eine Dorsalflexion zu bringen, also die Zehen anzuheben. Dadurch wird die Stolpergefahr deutlich verringert und ein flüssigeres und der natürlichen Bewegung entsprechenderes Treppensteigen wird ermöglicht.

Die mit der orthopädietechnischen Einrichtung versehene und mit ihr versorgte Extremität wird als ipsilaterale Extremität bezeichnet. Eine nicht mit der orthopädietechnischen Einrichtung versehene Extremität wird hingegen als kontralaterale Extremität bezeichnet. Dabei kann die kontralaterale Extremität zu der ipsilateralen Extremität korrespondieren, wenn beide Extremitäten beispielsweise Arme oder Beine sind. Ein nicht durch die orthopädietechnische Einrichtung versorgter Arm wird jedoch auch dann als kontralateral bezeichnet, wenn die ipsilaterale Extremität ein Bein ist und umgekehrt. Dies gilt im Sinne der vorliegenden Erfindung bevorzugt auch dann, wenn beide auf der gleichen Körperseite liegen, es sich also um einen linken Arm und ein linkes Bein oder einen rechten Arm und ein rechtes Bein handelt.

Herkömmlicherweise werden über den wenigstens einen Sensor Messwerte aufgenommen, aus denen Parameter der ipsilateralen Extremität bestimmt werden können. So kann beispielsweise der Kniewinkel, der Knöchelwinkel, verschiedene Momente, Relativpositionen unterschiedlicher Bauteile zueinander oder Geschwindigkeiten, Beschleunigungen oder Verlagerungen bestimmter Punkte der orthopädietechnischen Einrichtung relativ zueinander oder absolut bestimmt werden. All diese Parameter können verwendet werden, um den Bewegungszustand zu ermitteln. Nachteilig ist jedoch, dass der Bewegungszustand erst dann ermittelt werden kann, wenn die Messwerte erfasst wurden, also erst nach oder in dem jeweiligen Schritt. Die Bestimmung des Bewegungszustandes ist daher immer erst rückwirkend möglich. Eine Steuerung des steuerbaren Aktuators in Abhängigkeit des erkannten Bewegungszustandes basiert dabei immer auf der Voraussetzung, dass der Bewegungszustand des Trägers sich zwischen den beiden Schritten nicht ändert. Der in einem Schrittzyklus erkannte Bewegungszustand wird als auch für den nächsten Schrittzyklus gültig angesehen. Nachteile treten dann auf, wenn dies nicht der Fall ist und sich der Bewegungszustand ändert. Dies hat zur Folge, dass Träger von orthopädietechnischen Einrichtungen, beispielsweise Knie- und Unterschenkelprothesen das Treppensteigen immer mit der kontralateralen Extremität beginnen. Dies hat teilweise zur Folge, dass Träger, die eine Treppe steigen möchten, vor der Treppe das Standbein wechseln müssen, um mit dem für sie richtigen Fuß, also der kontralateralen Extremität, das Treppensteigen zu beginnen. Dies ist umständlich und unbequem und sorgt zudem dafür, dass ein Träger einer orthopädietechnischen Einrichtung sehr leicht als solcher erkannt werden kann. Die hervorzurufende Illusion einer natürlichen Bewegung ist auf diese Weise nicht oder nur sehr schwer aufrechtzuerhalten. Der vorliegenden Erfindung liegt daher die Aufgabe zugrunde, ein Verfahren zum Betreiben einer orthopädietechnischen Einrichtung so weiterzuentwickeln, dass die Erkennung des Bewegungszustandes verbessert wird und die Bewegung, die der Träger der orthopädietechnischen Einrichtung mit der orthopädietechnischen Einrichtung ausführt, der natürlichen Bewegung möglichst gleicht.

Die Erfindung löst die gestellte Aufgabe durch ein Verfahren zum Betreiben einer orthopädietechnischen Einrichtung gemäß dem Anspruch 1.

Der Erfindung liegt die Erkenntnis zugrunde, dass insbesondere der menschliche Gang aber auch viele andere menschliche Bewegungen im Wesentlichen bestimmt sind durch die koordinierte Bewegung unterschiedlicher Körperteile, insbesondere zweier Extremitäten. So muss beispielsweise für die Durchführung eines Schrittes das Standbein die Bewegung des Körperschwerpunktes übernehmen und die Vorwärtsprogression erzeugen, während das Schwungbein die Positionierung des Fußes in einer Weise vollziehen muss, dass die Balance gehalten und ein effizienter Gewichtstransfer ermöglicht wird. Soll beispielsweise mit einer Handprothese ein Geländer gegriffen und sich daran abgestützt werden, hat dies auch Bewegungen der Schulter und gegebenenfalls des Brustkorbes zur Folge. Die Erfindung macht sich die Idee zu Nutze, die Zusammenhänge einer derartigen Kopplung auszunutzen, um die Bewegung der eingeschränkten ipsilateralen Extremität zumindest auch aus einer kontralateralen Bewegung der Extremität zu rekonstruieren und zu steuern. Dies gilt insbesondere, wenn auch nicht ausschließlich, für die Intention also das Erkennen des Bewegungszustandes zu einem möglichst frühen Zeitpunkt.

Der mindestens eine Aktuator der Orthese kann dabei beispielsweise so angesteuert werden, dass die Bewegung und/oder Unterstützung der versorgten Extremität, die in diesem Fall den ersten Körperteil bildet, in Abhängigkeit der Bewegung einer kontralateralen Extremität erfolgt, die in diesem Fall den zweiten Körperteil bildet. Beispielsweise kann bei einer Arm-Orthese durch den mindestens einen Sensor eine Beinstreckung aus einer eingebeugten Stellung erkannt werden, welche auf eine Hebebewegung schließen lässt. Die Orthese wird dann derart aktuiert, dass die Orthese ein Anheben der Arme und/oder eine Streckbewegung unterstützt und/oder durchführt. Es ist allerdings auch möglich, dass es sich bei der Orthese um eine Unterstützung der Lendenwirbelsäule handelt, die den ersten Körperteil bildet und deren Nachgiebigkeit durch einen Aktuator verstellt werden kann. Die Nachgiebigkeit wird beispielsweise verändert, wenn eine Streckbewegung der Beine aus einer eingebeugten Stellung und/oder ein Einbeugen der Ellenbogen aus einer gestreckten Stellung, detektiert wird, insbesondere dann, wenn der Oberkörper nach vorne geneigt ist. In beiden Fällen wird die Ansteuerung des Aktuators an die Bewegung zumindest eines zweiten Körperteils gekoppelt.

Erfindungsgemäß wird der zeitliche Verlauf eines Parameters zum Erkennen des Bewegungszustandes verwendet. Eine Einzelmessung, die einen Messwert zu einem einzelnen Zeitpunkt liefert, ist nicht ausreichend. Es ist von Vorteil, jedoch nicht notwendig, wenn der zeitliche Verlauf des Parameters insbesondere bei sich wiederholenden Bewegungen über einen oder besonders bevorzugt mehrere Zyklen, beispielsweise Schrittzyklen bestimmt wird. Dies geschieht in der Regel durch eine Mehrzahl, vorzugsweise eine Vielzahl von Einzelmessungen, die den Messwert zu jeweils einem einzelnen Zeitpunkt liefern. Diese Ergebnisse der Einzelmessungen werden gespeichert und als zeitlicher Verlauf ausgewertet. Die Mehrzahl von Einzelmessungen kann dabei zeitlich äquidistant erfolgen. Der Abstand zwischen zwei Einzelmessungen muss dabei klein gegenüber der Länge beispielsweise eines Schrittzyklus sein, sodass ein zeitlicher Verlauf des Parameters aus der Mehrzahl der Einzelmessungen ermittelt werden kann.

Oftmals ist es von Vorteil und ausreichend, den zeitlichen Verlauf des Parameters nicht über ganze Schrittzyklen, sondern beispielsweise nur über bestimmte Teile eines Schrittzyklus zu bestimmen. Zum Erkennen eines Bewegungszustandes ist es oftmals ausreichend, den Parameter zu ganz bestimmten Zeitpunkten beispielsweise eines Schrittzyklus zu kennen. Diese bestimmten Zeitpunkte können beispielsweise der Fersenauftritt auf dem Boden oder der Zeitpunkt des Ablösens der Zehen sein. Um diesen Zeitpunkt möglichst exakt zu ermitteln ist es notwendig oder mindestens vorteilhaft, den zeitlichen Verlauf des Parameters in einem bestimmten Zeitraum vor und nach diesem bestimmten Zeitpunkt zu messen und zu bestimmen. Auch dies fällt unter die erfindungsgemäße Definition eines zeitlichen Verlaufes.

Ist der Bewegungszustand des Trägers, also insbesondere die Art seines Fortbewegens, erkannt worden, kann der steuerbare Aktuator entsprechend gesteuert werden. Dabei werden bevorzugt nicht einfache Routinen und zeitliche Abläufe, die für bestimmte Bewegungszustände in einem Datenspeicher einer elektronischen Steuereinheit hinterlegt sind, ausgeführt. Vielmehr wird bevorzugt der zeitliche Verlauf des Parameters des zweiten Körperteiles zur Steuerung des wenigstens einen steuerbaren Aktuators verwendet. Auf diese Weise wird erreicht, dass der durch die orthopädietechnische Einrichtung unterstützte oder ersetzte Körperteil harmonisch, natürlich und möglichst optimal an die Bewegungen der anderen Körperteile, insbesondere des zweiten Körperteils, angepasst bewegt wird. So wird beispielsweise ein natürliches Gangbild erzeugt, indem beispielsweise die Bewegung einer orthopädietechnischen Einrichtung, beispielsweise eines Prothesenschaftes an die Bewegung eines gesunden Beines, das in diesem Fall das zweite Körperteil bildet, angepasst wird. Alternativ oder zusätzlich dazu kann das zweite Körperteil auch ein Arm sein, dessen natürliche Schwenkbewegung beim Gehen oder Laufen verwendet wird, um die Bewegung einer Beinprothese oder einer Orthese zu steuern.

Mit dem erfindungsgemäßen Verfahren ist es in besonders bevorzugten Ausgestaltungen folglich möglich, nicht nur den Bewegungszustand des Trägers möglichst frühzeitig zu erkennen und den wenigstens einen steuerbaren Aktuator entsprechend zu steuern, sondern die Bewegung des Aktuators an die Bewegung unterschiedlicher Körperteile anzupassen und so die Akzeptanz der orthopädietechnischen Einrichtung beim Träger zu vergrößern.

Vorzugsweise grenzt der erste Körperteil nicht direkt an den zweiten Körperteil an.

In einer bevorzugten Ausgestaltung ist der wenigstens eine Parameter eine Relativposition, Relativbewegung und/oder Relativgeschwindigkeit und/oder Relativbeschleunigung und/oder ein Relativwinkel des zweiten Körperteiles zu dem ersten Körperteil und/oder eines ersten Teils des zweiten Körperteiles zu einem zweiten Teil des zweiten Körperteils. Vorzugsweise handelt es sich bei dem zweiten Körperteil um einen Fuß, ein Knie, einen Oberschenkel, einen Unterschenkel und/oder eine Beinsehne. Das zweite Körperteil ist vorzugsweise eine unversorgte Extremität oder in Teil davon. Es kann jedoch auch von Vorteil sein, wenn das zweite Körperteil beispielsweise ein Teil einer Extremität ist, an der die orthopädietechnische Einrichtung angeordnet ist. So kann das zweite Körperteil beispielsweise ein Oberschenkel oder der Amputationsstumpf eines Beines sein, an dem eine Prothese angeordnet ist, deren künstliches Knie oder künstlicher Fuß beispielsweise das erste Körperteil ersetzt.

Um den zeitlichen Verlauf der Relativbewegung zu bestimmen, werden beispielsweise die Position der Körperteile zueinander und/oder ihre Lage in einem gemeinsamen Koordinatensystem relativ zu mindestens einem Sensor zu mehreren Zeitpunkten ermittelt. Unter Position wird insbesondere auch die translatorische und/oder rotatorische Orientierung zueinander verstanden werden.

Dabei ist es unerheblich, ob das zweite Körperteil, insbesondere die kontralaterale Extremität ebenfalls durch eine weitere orthopädietechnische Einrichtung versorgt ist.

Besonders interessant ist die Beinsehne eines unversorgten Beines als zweites Körperteil, die die gedachte Verbindungslinie zwischen einem Fuß und einer Hüfte der Extremität darstellt. Interessante Messgrößen der Beinsehne sind dabei insbesondere ihre Orientierung sowie ihre Länge sowie deren Geschwindigkeiten und Veränderungen. Die Beinsehne gibt einerseits Informationen über die Position des Fußes der kontralateralen Extremität in Relation zum Körperzentrum und zum Schwerpunkt. Sie gibt damit direkt und indirekt Informationen über die Progression und die Stabilität und/oder die Fußpositionierung des Trägers. Zudem ist die Bewegung der Beinsehne mit herkömmlichen Sensoren bereits zugänglich, auch wenn sie in der Regel nicht verwendet wird. Die Bewegung des proximalen Endpunktes der Beinsehne, also der Hüfte, kann bereits über vorhandene Sensorik, die in eine hier beschriebene orthopädietechnische Einrichtung integriert sein kann, berechnet werden. Über die Bewegung des distalen Endpunktes, also des Fußes der unversorgten Extremität, lassen sich insbesondere in der Standphase gute Annahmen treffen. **In** der Schwungphase kann die Bewegung, also insbesondere die Position und/oder die Änderung der Position, des Fußes durch den wenigstens einen Sensor bestimmt werden.

Sind der proximale Endpunkt und der distale Endpunkt der Beinsehne des unversorgten Beines bekannt, kann beispielsweise unter Zuhilfenahme bekannter Abmessungen des Oberschenkels und des Unterschenkels des Trägers der orthopädietechnischen Einrichtung auch ein Beinwinkel oder Kniewinkel bestimmt werden, der sich intuitiv interpretieren lässt und in Steuergesetzen verwendbar ist. Der Kniewinkel der versorgten Seite ist ein bewährter Steuerparameter.

Alternativ oder zusätzlich dazu kann bei einem mit der orthopädietechnischen Einrichtung versorgten Bein, also der ipsilateralen Extremität, auch die Position des kontralateralen, also unversorgten, Fußes in Relation zum ipsilateralen Fuß bestimmt werden. Dies kann entweder ausschließlich innerhalb der Sagittalebene oder dreidimensional erfolgen. Nachdem in vielen Situationen davon ausgegangen werden kann, dass wenigstens einer der Füße Bodenkontakt hat, kann eine relative Messung des Abstandes zwischen dem ipsilateralen Fuß und dem kontralateralen Fuß als Bestimmung einer absoluten Trajektorie angesehen werden. Die direkte Positionsmessung ist dabei wesentlich zuverlässiger als die zweifache Integration von Beschleunigungsmessungen, nicht zuletzt aufgrund der Notwendigkeit von korrekten Anfangsbedingungen bei der Integration. Selbstverständlich ist es auch möglich, Beschleunigungen und wirkende Momente auf einen Fuß zu bestimmen und diese in Form einer Messreihe oder einem zeitlichen Verlauf darzustellen. Die zweifache Integration über die Zeit ergibt die Bewegung. Die horizontale Komponente der Fußbewegung gibt Aufschluss über die Schrittlänge und damit auch das Timing eines Schrittes. Dabei ist insbesondere der Moment interessant, wenn der kontralaterale Fuß den ipsilateralen Fuß passiert. Dies gilt sowohl in der Stand- als auch in der Schwungphase.

Auch die relativen Positionen anderer Punkte relativ zueinander, beispielsweise die ipsilaterale Knieachse zum kontralateralen Fuß, kann von Interesse sein. Je mehr Sensoren verwendet werden, desto mehr unterschiedliche Parameter sind einer Messung zugänglich. Über kinematische Ketten kann auf andere Relativpositionen zurückgeschlossen werden, so dass weitere Parameter zugänglich werden.

Insbesondere aus den Relativpositionen und/oder Relativbewegungen sowie die Relativwinkeln in verschiedenen Kombinationen kann auch auf Segmentwinkel der kontralateralen Seite zurückgeschlossen werden. Dies betrifft beispielsweise den Oberschenkel, den Unterschenkel oder den Fuß. Daraus können Gelenkswinkel, beispielsweise der kontralaterale Hüftwinkel, der Kniewinkel oder der Knöchelwinkel bestimmt werden.

Durch die geschickte Wahl unterschiedlicher Sensoren zur Bestimmung unterschiedlicher Größen, aus denen die verschiedenen Parameter auch des zweiten Körperteiles bestimmt werden können, kann beispielsweise auf die kontralaterale Beinbewegung geschlossen werden.

Bevorzugt ist der wenigstens eine Sensor eingerichtet, einen Absolutwinkel, einen Relativwinkel, eine Geschwindigkeit, eine Beschleunigung, eine Kraft, einen Druck, eine Druckwelle, ein Moment, ein elektrisches Feld und/oder ein magnetisches Feld zu detektieren. Unter einer Druckwelle wird insbesondere auch eine Schallwelle, insbesondere eine Ultraschallwelle verstanden.

Vorzugsweise ist das erste Körperteil eine ipsilaterale Extremität oder ein Teil davon, insbesondere ein Fuß, ein Knöchel und/oder ein Knie, und das zweite Körperteil eine andere Extremität, vorzugsweise eine kontralaterale Extremität, oder ein Teil davon, bevorzugt ein Fuß, ein Knöchel und/oder ein Knie.

In einer bevorzugten Ausgestaltung ist der wenigstens eine Sensor an einem Bauteil der orthopädietechnischen Einrichtung und/oder an der dem ersten Körperteil angeordnet und detektiert vorzugsweise zumindest auch Messwerte, aus denen der wenigstens eine Parameter des zweiten Körperteils, insbesondere der kontralateralen Extremität bestimmt wird. Vorzugsweise handelt es sich bei dem wenigstens einen Sensor um einen berührungslosen Sensor. Dabei stehen unterschiedliche Messprinzipien zur Auswahl. Der wenigstens eine Sensor kann beispielsweise Informationen über die kontralaterale Extremität durch die Messung einer Beeinflussung von elektrischen, magnetischen und/oder elektromagnetischen und elektrostatischen Feldern bestimmen. Dies gilt beispielsweise durch die Beeinflussung von Schwingkreisen oder durch kapazitive Messungen. Derartige Sensoren sind aus dem Stand der Technik dem Fachmann bekannt, so dass auf eine detaillierte Beschreibung verzichtet wird.

Ein anderes Wirkprinzip einer berührungslosen Messung ist beispielsweise die Bestimmung von Ausbreitungszeiten, Reflexionen und Interferenzen von Wellen, die vorteilhafterweise vom Sensor selbst oder einem anderen Bauteil, das an der orthopädietechnischen Einrichtung und/oder der ipsilateralen Extremität angeordnet ist, ausgesandt werden.

Vorzugsweise ist der wenigstens eine Sensor an dem zweiten Körperteil, bevorzugt an der kontralateralen Extremität angeordnet ist und vorzugsweise zumindest auch Messwerte detektiert, aus denen der wenigstens eine Parameter des funktionalen Körperteil, bevorzugt der kontralateralen Extremität bestimmt wird.

Erfindungsgemäß verfügt der wenigstens eine Sensor daher über wenigstens eine Sendeeinrichtung und wenigstens eine Empfangseinrichtung. Die Sendeeinrichtung sendet dabei eine Messstrahlung aus, bei der es sich vorteilhafterweise um Ultraschall-Wellen oder eine elektromagnetische Messstrahlung, beispielsweise Radarstrahlung und/oder sichtbares Licht und/oder IR-Strahlung handelt. Die Empfangseinrichtung ist eingerichtet, diese Messstrahlung zu empfangen. Mit einem derartigen Sensor sind die Prinzipien der Interferenzmessung, Triangulation und Laufzeitmessung unterschiedlicher Elektromagneten oder anderen Messstrahlungen zugänglich.

Als Messstrahlungen kommen elektromagnetische Wellen im Radio- und Mikrowellenbereich, beispielsweise Radar, nahe und ferne Infrarotstrahlung und sichtbares Licht, beispielsweise LIDAR in Frage. Eine entsprechende Empfangseinrichtung ist für sichtbares Licht beispielsweise eine Kamera. Handelt es sich bei der Messstrahlung nicht um eine elektromagnetische Strahlung, kann beispielsweise eine Ultraschallstrahlung verwendet werden. Die vom Sender ausgesandte Messstrahlung trifft auf das zweite Körperteil, bevorzugt die kontralaterale Extremität und wird von ihr beeinflusst. Dies betrifft einerseits die Reflexion der Messstrahlung aber auch eine Veränderung der Frequenz und insbesondere der Phasen ist möglich. Die Empfangseinrichtung ist eingerichtet, diese von dem zweiten Körperteil beeinflusste Messstrahlung zu empfangen und die enthaltenen Informationen auszuwerten.

Erfindungsgemäß ist die Empfangseinrichtung eingerichtet, Messstrahlung, die von dem zweiten Körperteil, bevorzugt der kontralateralen Extremität reflektiert oder reemittiert wurde, zu empfangen und aus einer Laufzeit, einer Phasenverschiebung, einer Frequenzverschiebung und/oder einer Interferenz mit der ausgesandten Messstrahlung die Messwerte und daraus den wenigstens einen Parameter zu bestimmen. Die Bestimmung des Parameters und die Auswertung der Messwerte erfolgt dabei vorteilhafterweise nicht in der Empfangseinrichtung, sondern in der elektrischen Steuerung, die auch zur Steuerung des steuerbaren Aktuators verwendet wird.

Über diese Methoden können Orientierungen, Abstände, Positionen und gegebenenfalls auch Geschwindigkeiten ermittelt werden. Insbesondere bei der Geschwindigkeitsbestimmung wird beispielsweise der Dopplereffekt verwendet. All diese Methoden und Auswerteverfahren können dabei sowohl in zweidimensionalen, beispielsweise in er Sagittalebene, als auch im Dreidimensionalen verwendet werden. Zusätzlich können aus dem Stand der Technik prinzipiell bekannte Techniken der Bilderkennung benutzt werden, um Objekte, insbesondere die kontralaterale Extremität oder Teile der kontralateralen Extremität zu bestimmen.

Auch Photogrammetrie oder Lichtschnittmethoden können verwendet werden, um beispielsweise Tiefeninformationen aus den Messwerten des wenigstens einen Sensors zu extrahieren. All diese Verfahren werden vorzugsweise in der elektrischen Steuerung verwendet.

Die Messung selbst kann punktuell, in einer definierten Ebene oder in einem Richtungsbereich, beispielsweise einem Sendekegel, erfolgen. Dabei ist es möglich, die gesamte Szenerie in einer einzigen Aufnahme abzudecken oder jeweilige Rasterungen durchzuführen. Dabei kann ausgenutzt werden, dass sich der wenigstens eine Sensor an dem ersten Körperteil oder einem Bauteil der orthopädietechnischen Einrichtung an dem zweiten Körperteil, insbesondere der kontralateralen Extremität vorbeibewegt oder umgekehrt. Das zweite Körperteil wird daher von dem wenigstens einen Sensor in unterschiedlichen Perspektiven erfasst, so dass unterschiedliche Informationen enthalten sind.

Sender und Empfänger sind bevorzugt an demselben Körperteil, beispielsweise einem ipsilateralen oder kontralateralen Körperteil angebracht. In anderen Ausgestaltungen ist es auch möglich, Sender und Empfänger auf jeweils unterschiedlichen Körperteilen befinden. Des Weiteren ist bei mehreren Sensoren auch eine Kombination von Anordnungen auf demselben sowie anderem Körperteil möglich.

In einer bevorzugten Ausgestaltung ist an dem zweiten Körperteil, bevorzugt der kontralateralen Extremität wenigstens ein Transponder und/oder ein Tag und/oder ein Reflektor für die ausgesandte Messstrahlung angeordnet. Dabei handelt es sich um ein sogenanntes Target, welches aufgrund von geometrischer Form und/oder Materialeigenschaften für die entsprechende Sensortechnologie und elektromagnetische Strahlung leicht identifizierbar ist und klar definierte Eigenschaften besitzt. Auch aktive und passive Transponder können verwendet werden, um beispielsweise Informationen zur Identifikation oder eigenständige Messergebnisse auszusenden, sobald sie von der Messstrahlung getroffen werden. Ein derartiger Transponder oder Target kann beispielsweise in einem Band oder einen Gurt integriert sein, der beispielsweise an dem zweiten Körperteil angeordnet oder in einem Kleidungsstück positioniert ist.

Es hat sich als vorteilhaft herausgestellt, wenn zum Bestimmen des wenigstens einen Parameters, insbesondere zum Bestimmen des wenigstens einen Parameters des zweiten Körperteils, bevorzugt der kontralateralen Extremität Daten der orthopädietechnischen Einrichtung und/oder des Trägers verwendet werden. Dies können beispielsweise Entfernungen, mögliche Schwenkwinkel oder Längenangaben sein. So ist es beispielsweise zur Bestimmung eines Kniewinkels eines kontralateralen Beins aus der Beinsehne notwendig, zumindest grob, vorzugsweise jedoch genau, die Unterschenkellänge und die Oberschenkellänge des Trägers der orthopädietechnischen Einrichtung auf der kontralateralen Seite zu kennen. Relative Größen der kontralateralen Seite zur ipsilateralen Seite können durch Messung von absoluten Messgrößen der ipsilateralen Seite ebenfalls in absolute Größen umgerechnet werden. So entspricht beispielsweise ein kontralateraler Unterschenkelwinkel dem ipsilateralen Unterschenkelwinkel plus dem Relativwinkel der beiden Unterschenkel zueinander.

Vorzugsweise wird beim Betreiben der orthopädietechnischen Einrichtung wenigstens eine Steuergröße des wenigstens einen steuerbaren Aktuators auf einen Sollwert oder einen Sollwertverlauf gesteuert. Dieser ist vorteilhafterweise nicht nur vom erkannten Bewegungszustand selbst, sondern auch von dieser Erkennung zugrunde liegenden Parametern insbesondere von dem wenigstens einen Parameter des zweiten Körperteils, bevorzugt der kontralateralen Extremität abhängig.

Die Erfindung löst die gestellte Aufgabe zudem durch eine orthopädietechnische Einrichtung, die ein erstes Körperteil unterstützt oder ersetzt, wobei die orthopädietechnische Einrichtung wenigstens einen Sensor und eine elektrische Steuerung aufweist, die eingerichtet ist zum Durchführen eines hier beschriebenen Verfahrens.

Bei der Bestimmung des wenigstens einen Parameters der kontralateralen Extremität kann, wie bereits dargelegt, auf Berechnungen des entsprechenden Parameters aus Sensordaten zurückgegriffen werden. Alternativ oder zusätzlich dazu können fehlende Parameter, die mit den verwendeten Sensoren nicht direkt zugänglich sind, aus vorhandenen Messgrößen und gegebenenfalls einem Model oder Modelannahmen bestimmt werden. Die vorhandenen Messgrößen können dabei sowohl Messgrößen der ipsilateralen als auch der kontralateralen Seite sein. Entsprechende Modelle sind beispielsweise mechanische und kinematische Modelle, die die jeweiligen Bewegungen der Extremität beschreiben.

Ein Beispiel für eine Anwendung eines Verfahrens gemäß einem Ausführungsbespiel der vorliegenden Erfindung sieht vor, dass der Beugewiderstand einer Knieprothese oder einer knieübergreifenden Orthese reduziert wird. Das Knie bildet den ersten Körperteil. Diese Reduzierung oder die Schwungphasen-Freischaltung findet in der ipsilateralen Standphase in Abhängigkeit des Beinwinkels und/oder der Segmentwinkel der kontralateralen Schwungbeinphase statt. Sie kann zumindest teilweise auch beim Treppab- und Bergab-Gehen erfolgen. Die Reduzierung geschieht so, dass eine Reduktion dann, oder erst dann, erfolgt wenn der kontralaterale Fuß, also der zweite Körperteil, hinreichend nahe zum ipsilateralen Fuß, also dem Fuß am ersten Körperteil, steht oder diesen bereits in anteriore Richtung passiert hat. Eine solche gezielte Reduktion des Beugewiderstands ermöglicht es, den Beugewiderstand in der frühen Standphase höher als derzeit zu gestalten oder nach einem gewissen Ausmaß an Kniebeugung eine weitere Beugung zu unterbinden und erst wieder zuzulassen wenn der ipsilaterale Fuß ausreichend weit nach vorne geschwungen ist. Der Zeitpunkt der Reduktion sowie der initiale Beugewiderstand können zudem von der Gehgeschwindigkeit abhängen, wobei eine höhere Gehgeschwindigkeiten zu einer geringeren Überhöhung des Beugewiderstands und einer früheren Reduktion führt. Für einen Prothesenfuß oder eine fußübergreifende Orthese kann die Dorsalflexionsbewegung und/oder der Widersand gegen eine Dorsalflexion in der ipsilateralen Standphase angepasst werden, um ein leichtes Überrollen zu ermöglichen. Insbesondere kann durch ein Schwingen der kontralateralen Seite nach vorn aus stehender Position erkannt werden, dass ein Vorwärtsschritt eingeleitet wird und gegenüber dem Stehen eine Dorsalflexion zugelassen und/oder eingeleitet werden, welches das Abrollen über den Fuß erleichtert.

In einem anderen Ausführungsbeispiel wird aus dem relativen Abstand des ipsilateralen Fußes zum kontralateralen Fuß durch den Bodenkontakt der jeweils gegenüberliegenden Seite direkt die Trajektorie des ipsilateralen sowie kontralateralen Fußes in der jeweiligen Schwungphase bestimmt werden. Der ipsilaterale Fuß bildet den ersten Körperteil, der kontralaterale Fuß bildet den zweiten Körperteil. Im Fall einer trans-tibialen Versorgung oder einer Knöchel-Fuß-Orthese (AFO - ankle foot orthosis) gibt diese Aufschluss über die zu überwindende Höhendifferenz. Dabei kann es sich sowohl um eine positive, also entgegen der Schwerkraft gerichteten Höhendifferenz handeln, als auch eine negative, also ein nach unten Steigen. Das Hilfsmittel daraufhin so gesteuert, dass der Fuß seine Neigung oder Steifigkeit vor Initialkontakt an die Situation optimal anpasst. Insbesondere ist es möglich bei einem nach unten Steigen den vorauseilenden Fuß in stärkere Plantarflexion zu bringen um bei Initialkontakt mit dem Vorfuß aufzusteigen. Auch ist es möglich, dass bei einem nach oben Steigen der kontralateralen Seite, der ipsilaterale Fuß in dessen Standphase eine aktive Plantarflexion durchführt um den Körperschwerpunkt anzuheben und das Überwinden einer Höhendifferenz zu erleichtern.

Für knieübergreifende Versorgungen kann neben der relativen Position der Füße zueinander auch die Relation der Bewegung des Schwungbeins und des Standbeins in Relation gesetzt werden, insbesondere der ipsilateralen und kontralateralen Beinsehne. In diesem Fall werden mehrere zweite Körperteile verwendet. Aus dem Verhältnis der Bewegungen kann der Bewegungszustand ermittelt werden, insbesondere das Überwinden einer Höhendifferenz. Insbesondere beim nach oben Steigen mit der ipsilateralen Seite, kann das Kniegelenk in der Schwungphasen-Beugung stärker einbeugt werden und/oder in gebeugter Stellung am Ende der Schwungphasen-Streckung abgestoppt werden. Dies erleichtert das Überwinden einer Höhendifferenz. Es ist auch möglich, dass die Knieprothese oder knieübergreifende Orthese in der Schwungphase so gesteuert wird, dass die Bewegung des ipsilateralen Fußes in einem harmonischen Verhältnis zur Bewegung des kontralateralen Beines sowie der Bewegung des ispislateralen Oberschenkels oder Oberschenkel-Stumpfes steht. Beispielsweise kann das Kniegelenk so gesteuert werden, dass die Schrittlänge des vorauseilenden ipsilateralen Fußes in etwa jener des kontralateralen Standbeins entspricht. Eine stärkere ipsilaterale Hüftflexion bei gleichbleibender kontralateraler Beinbewegung kann beispielsweise zu einer geringeren Knie-Extension oder stärkeren Knie-Flexion führen.

In einem anderen Beispiel wird die Knie-Vorflexion eines Knies angepasst. Wird das Kniegelenk beim Aufwärtsgehen auf Rampen und Treppen sowie beim Hochsteigen am Ende der Schwungphase in einer flektierten Stellung abgestoppt kann das Ausmaß dieser Vorflexion so bestimmt werden, dass ipsilateraler und kontralateraler Beinwinkel bei ipsilateralem Initialkontakt in einem harmonischen Verhältnis zueinander stehen. Der Anwender bestimmt die Schrittlänge damit im Wesentlichen über die kontralaterale Standbeinbewegung und die Schritthöhe über die ipsilaterale, hilfsmittelseitige Hüftflexion, respektive Oberschenkelbewegung.

Auch eine Schwungphasen-Steuerung ist möglich. Die Flexions- und Extensionswiderstände, bzw. die Sollwerte eines Aktuators, in der prothesenseitigen Schwungphase könnten so eingestellt werden, dass sich ein harmonisches Verhältnis zwischen Beinwinkel der kontralateralen Seite in deren Standphase und jenem der ipsilateralen Seite in der Schwungphase einstellt. Die Abrollbewegung der kontralateralen Seite würde damit das Timing der ipsilateralen Seite vorgeben, wobei die ipsilaterale Oberschenkelbewegung einen wesentlichen Einfluss darauf hat, wie die orthopädietechnische Einrichtung in die Bewegung eingreifen muss.

In einem anderen Beispiel wird die Schwungbewegung erkannt. Manche Hilfsmittel verfügen über keinerlei Kraftsensorik um festzustellen, ob sich die versorgte Seite in Bodenkontakt befindet. Die kontralaterale Beinbewegung kann Aufschluss darüber geben, ob es sich um ein nach hinten Gehen handelt, bei der das kontralaterale Standbein nach hinten abrollt, oder ob das Standbein still steht und die ipsilaterale Seite unter dem Körper nach hinten geschwungen wird. In letzterem Fall kann bei knieübergreifenden Versorgungen eine Kniebeugung zugelassen oder eingeleitet werden, welche das Treppaufsteigen oder Übersteigen von Hindernissen ermöglicht. Ein ähnlicher Ansatz kann helfen zu erkennen, wenn der Anwender die ipsilaterale Seite nach vorne setzt, zum Beispiel aus dem Stehen.

Ein wichtiger Anwendungsfall ist die Stolper-Erkennung. Die Information über die kontralaterale Beinbewegung kann ebenfalls Aufschluss darüber geben, ob die Anwenderin stolpert. Dies betrifft sowohl ein Stolpern in der ipsilateralen als auch in der kontralateralen Schwungbeinphase. Eine Erkennung kann einerseits durch das abrupte Abstoppen der ansonsten kontinuierlichen Bewegung erfolgen oder aber durch ein zu starkes Nacheilen der Schwungbeinseite in Relation zur Abrollbewegung der gegenüberliegenden Seite. Die Art der Reaktion bei detektiertem Stolpern kann im Weiteren davon abhängen, wo sich die gegenüberliegende Seite gerade befindet. Es ist sowohl ein Anheben des Fußes und/oder ein Erhöhen der Bodenfreiheit als auch ein Absetzen des Fußes und/oder eine Erhöhung des Beugewiderstands möglich.

Bei messtechnischer Erfassung des relativen Abstands von kontralateralem und ipsilateralem Fuß steht die Schrittlänge direkt zu Verfügung und kann neben der Steuerung für das Activity-Tracking oder die Beurteilung von Gangsymmetrie verwendet werden. Auch kann die Gehgeschwindigkeit direkt als pro Zeit zurückgelegter Weg bestimmt werden, anstatt sie aus der Abrollgeschwindigkeit in der ipsilateralen Standphase zu schätzen.

Mit Hilfe der beiliegenden Zeichnungen werden nachfolgend einige Ausführungsbeispiele näher erläutert. Es zeigen:
- Figur 1 -: vier unterschiedliche orthopädietechnische Einrichtungen jeweils in einer Frontalansicht,
- Figur 2 -: eine getragene orthopädietechnische Einrichtung in einem Gehmodus,
- Figur 3 -: die orthopädietechnische Einrichtung aus Figur 1 in einer schematischen Schnittdarstellung in drei unterschiedlichen Schrittpositionen,
- Figur 4 -: eine schematische Darstellung einer Anwendung eines hier beschriebenen Verfahrens,
- Figur 5 -: ein weiteres Beispiel einer Anwendung, sowie
- Figur 6 -: ein Flussdiagramm.

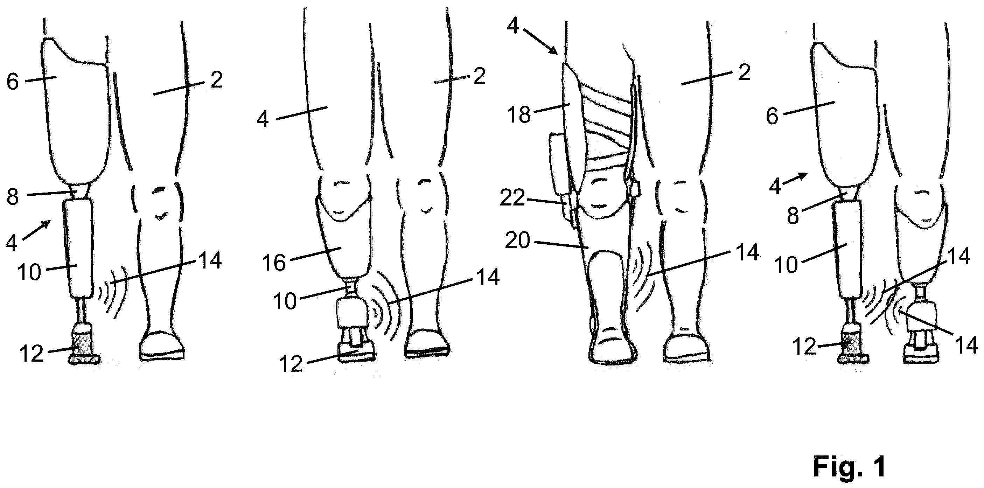

Figur 1 zeigt von links nach rechts vier unterschiedliche Versorgungssituationen. Ganz links sind die Beine eines Trägers einer orthopädietechnischen Einrichtung zu erkennen, bei dem die kontralaterale Extremität 2 das linke Bein ist, während die ipsilaterale Extremität 4 das rechte Bein ist. In der ganz linken Darstellungen der Figur 1 ist an der ipsilateralen Extremität 4 eine Beinprothese mit einem Oberschenkelschaft 6, einem Kniegelenk 8, einem Unterschenkel 10 und einem Fuß 12 angeordnet. Schematisch ist dargestellt, dass sich am Unterschenkel 10 ein Sensor befindet, der eine Messstrahlung 14 in Richtung auf die kontralaterale Extremität 2 aussendet.

In der Darstellung daneben ist die kontralaterale Extremität 2 wieder ein unversorgtes gesundes Bein, während an der ipsilateralen Extremität 4 diesmal eine Unterschenkelprothese angeordnet ist. Sie verfügt über einen Unterschenkelschaft 16, an dem sich der Unterschenkel 10 und der Fuß 12 anschließt. Auch hier ist ein Sensor angeordnet, der die Messstrahlung 14 in Richtung auf die kontralaterale Extremität aussendet.

Die dritte Darstellung von links zeigt eine gesunde kontralaterale Extremität 2 und eine vollständig vorhandene ipsilaterale Extremität 4, an der eine orthopädietechnische Einrichtung in Form einer Orthese angeordnet ist. Sie verfügt über einen Oberschenkelrahmen 18, einen Unterschenkelrahmen 20 und ein Kniegelenk 22, an dem sich ein steuerbarer Aktuator befindet. Auch hier ist im Unterschenkelbereich, also am Unterschenkelrahmen 20 der Sensor angeordnet, der die Messstrahlung 14 in Richtung auf die kontralaterale Extremität aussendet.

In der ganz rechten Darstellung der Figur 1 ist die ipsilaterale Extremität 4 wie in der ganz linken Darstellung gezeigt. Im Gegensatz zur ganz linken Darstellung ist jedoch die kontralaterale Extremität ebenfalls durch eine orthopädietechnische Einrichtung, nämlich eine Unterschenkelprothese entsprechend der in der zweiten Darstellung von links gezeigten orthopädietechnischen Einrichtung versorgt. Beide orthopädietechnischen Einrichtungen verfügen nun jeweils über einen Sensor, der Messstrahlung 14 in Richtung auf die jeweils andere Extremität aussendet. Für die orthopädietechnische Einrichtung, die in Figur 1 links, also am rechten Bein dargestellt ist, handelt es sich bei der gegenüberliegenden Seite um die kontralaterale Extremität, auch wenn sie durch eine zusätzliche orthopädietechnische Einrichtung versorgt ist.

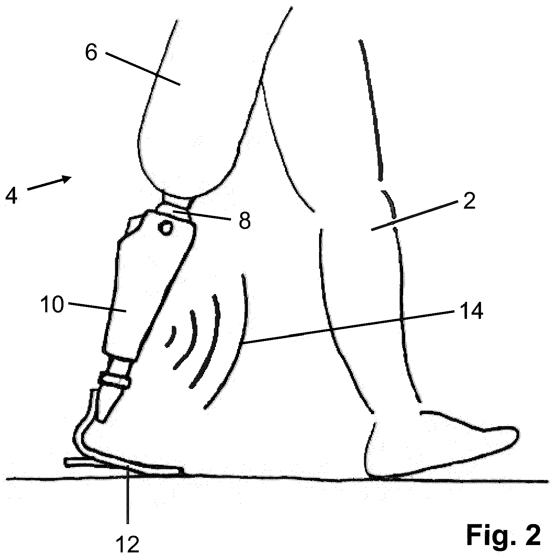

Figur 2 zeigt die Darstellung während eines Schrittzyklus. Die kontralaterale Extremität 2 ist unversorgt während sich an der ipsilateralen Extremität 4 eine Oberschenkelprothese mit Oberschenkelschaft 6, Kniegelenk 8, Unterschenkel 10 und Fuß 12 befindet. Während die Sensoren in Figur 1 die Messstrahlung 14 nach medial, also nahezu ausschließlich zur Seite ausgesandt haben, ist der Sensor in Figur 2 eingerichtet, die Messstrahlung 14 in Richtung auf die kontralaterale Extremität 2 zu senden, obwohl diese sich nahezu vollständig vor der ipsilateralen Extremität befindet. Dies kann erreicht werden, indem beispielsweise der Sendebereich, in den der Sensor die Messstrahlung 14 aussendet, so groß ist, dass unabhängig von der Position der kontralateralen Extremität 2 ausreichend Messstrahlung 14 die kontralaterale Extremität 2 trifft. Alternativ dazu kann der Sensor beziehungsweise insbesondere die Sendeeinrichtung gedreht oder verschoben werden. Alternativ oder zusätzlich dazu lässt sich auch die Abstrahlcharakteristik des entsprechenden Sensors anpassen.

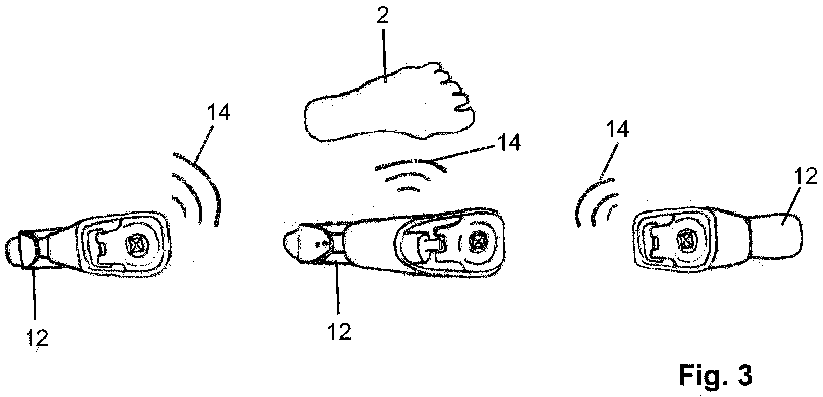

Dies ist in Figur 3 dargestellt. Man erkennt den Fuß der kontralateralen Extremität 2 sowie in einer abgeschnittenen Draufsicht den Fuß 12 der ipsilateralen Extremität 2 in verschiedenen Phasen eines Schrittes. Die ipsilaterale Extremität 4 führt gerade die Schwungphase durch, während der Fuß der kontralateralen Extremität 2 fest am Boden steht. In der ganz linken Darstellung der Figur 3 hat die ipsilaterale Extremität gerade den Bodenkontakt verloren und beginnt die Schwungphase. Die Messstrahlung 14 wird stark nach vorn gerichtet ausgesandt, da sich in dieser Richtung die translaterale Extremität befindet. In der Mitte der Schwungphase, die in der Mitte der Figur 3 dargestellt ist, befindet sich die ipsilaterale Extremität 4 direkt neben der kontralateralen Extremität, so dass die Messstrahlung 14 nahezu vollständig zur Seite ausgesandt wird. Am Ende der Schwungphase, die rechts in Figur 3 dargestellt ist, befindet sich der Fuß der ipsilateralen Extremität 4 vor dem Fuß der kontralateralen Extremität 2, so dass die Messstrahlung 14 zu einem Großteil nach hinten ausgesandt wird.

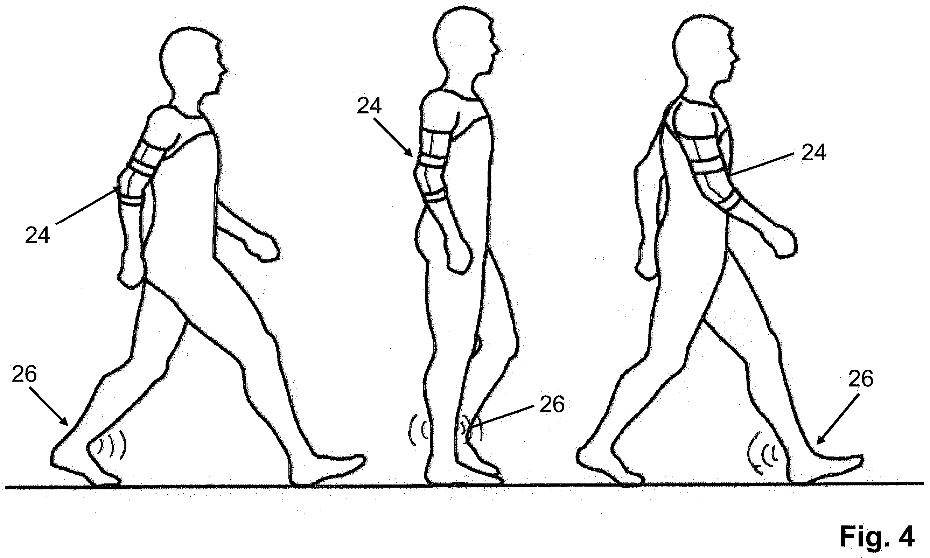

Figur 4 ist ein Beispiel dafür, dass der erste Körperteil 24, der im gezeigten Ausführungsbeispiel der rechte Arm des Trägers ist, nicht zwangsläufig "gegenüber" des zweiten Körperteils 26 liegen muss, der im gezeigten Ausführungsbeispiel der linken Knöchel ist. Figur 4 zeigt drei Positionen innerhalb eines Schrittzyklus, bei denen jeweils die Position des zweiten Körperteils 26, also des linken Knöchels, relativ zu einem weiteren Körperteil, nämlich dem rechten Knöchel, bestimmt wird. In der linken Darstellung der Figur 4 befindet sich der zweite Körperteil 26 hinter dem Rumpf des Trägers. Gleiches gilt für den ersten Körperteil 24. Die Relativposition des zweiten Körperteils 26 relativ zum rechten Knöchel wird bestimmt, was durch die drei kleinen Linien angedeutet wird. Im Verlauf eines Schrittzyklus verändert sich die Position des zweiten Körperteils 26 relativ zum rechten Knöchel über die in der Mitte der Figur 4 gezeigten Positionen innerhalb der Schwungphase bis zur in Figur 4 rechts dargestellte Position beim Fersenauftritt. Entsprechend wird auch die Bewegung des ersten Körperteils 24, das von einer Arm-Prothese ersetzt wird, gesteuert.

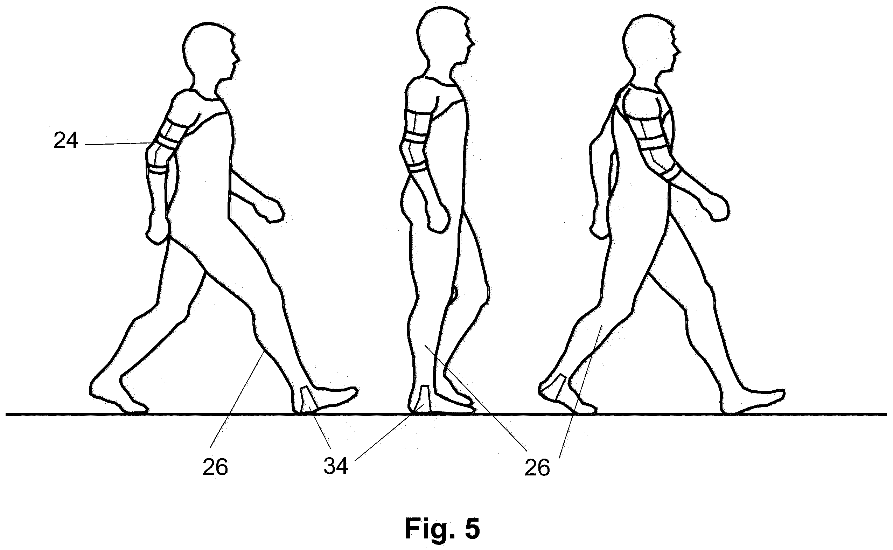

Figur 5 ist ein Beispiel dafür, dass sich das zweite Körperteil 26, auf dem ein Sensor 34 zur Bestimmung des Bewegungszustandes, insbesondere der Stand-Phase im Schrittzyklus, angebracht ist, auf der selben Körperhälfte befinden kann wie das Körperteil 24, das mit einem orthopädietechnischen Hilfsmittel ausgestattet ist. Dieser kann - wie beispielsweise im Falle eines Inertialsensors - ausschließlich aufgrund von Messungen der mit dem Sensor 34 ausgestatteten Extremität 26 Informationen über den Bewegungszustand erfassen. Ebenso können mittels des am Körperteil 26 angebrachten Sensors 34 auch Messstrahlen empfangen werden, die vom gegenüberliegenden Bein ausgesandt oder reflektiert oder reemittiert werden.

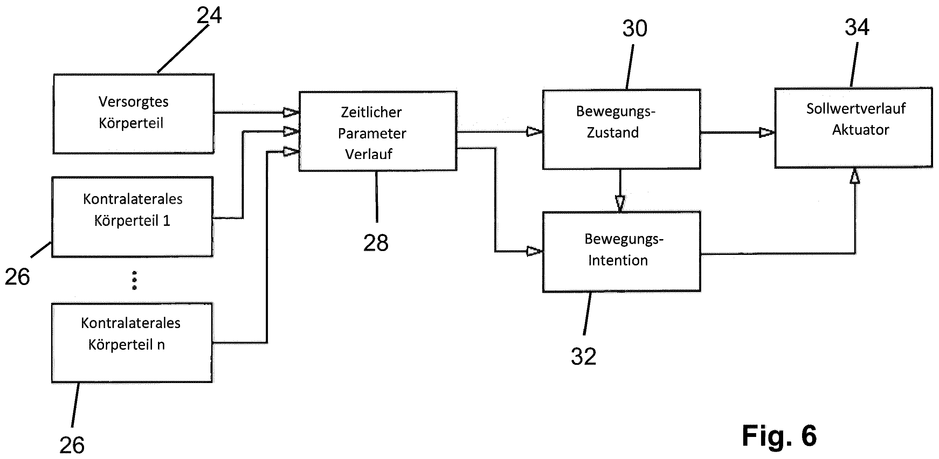

Figur 6 zeigt ein schematisches Flussdiagramm für ein hier beschriebenes Verfahren. Von einem ersten Körperteil 24 und mindestens einem zweiten Körperteil 26 werden Parameter ermittelt, deren zeitlicher Verlauf 28 bestimmt wird. Aus diesem wird sowohl ein Bewegungszustand 30 des Trägers als auch eine Bewegungsintention 32 bestimmt, wobei zur Bestimmung der Bewegungsintention 32 auch der bestimmte Bewegungszustand 30 herangezogen werden kann. Sowohl die Bewegungsintention 32 als auch der bestimmte Bewegungszustand 30 können getrennt voneinander oder in Kombination verwendet werden, um die Aktuator Steuerung 34 zu initiieren.

### Bezugszeichenliste

- 2: kontralaterale Extremität
- 4: ipsilaterale Extremität
- 6: Oberschenkelschaft
- 8: Kniegelenk
- 10: Unterschenkel
- 12: Fuß
- 14: Messstrahlung
- 16: Unterschenkelschaft
- 18: Oberschenkelrahmen
- 20: Unterschenkelrahmen
- 22: Kniegelenk
- 24: erster Körperteil
- 26: zweiter Körperteil
- 28: zeitlicher Verlauf
- 30: Bewegungszustand
- 32: Bewegungsintention
- 34: Sensor

## Patentansprüche

1. Verfahren zum Betreiben einer orthopädietechnischen Einrichtung, die
- ein erstes Körperteil (24) eines Trägers unterstützt oder ersetzt und
- wenigstens einen steuerbaren Aktuator aufweist,
wobei das Verfahren die folgenden Schritte aufweist:
a) Bestimmen eines zeitlichen Verlaufes (28) wenigstens eines Parameters, der eine Aussage über einen Bewegungszustand (30) des Trägers erlaubt, aus Messwerten wenigstens eines Sensors (34),
b) Erkennen des Bewegungszustandes (30) aus dem wenigstens einen bestimmten zeitlichen Verlauf (28) und
c) Steuern des wenigstens einen steuerbaren Aktuators in Abhängigkeit des erkannten Bewegungszustandes (30),
wobei zum Erkennen des Bewegungszustandes (30) zumindest auch der zeitliche Verlauf (28) wenigstens eines Parameters eines zweiten Körperteiles (26) des Trägers verwendet wird, wobei der wenigstens eine Sensor (34) wenigstens eine Sendeeinrichtung und wenigstens eine Empfangseinrichtung aufweist, wobei die Sendeeinrichtung eine Mess-Strahlung (14) aussendet und die Empfangseinrichtung eingerichtet ist, Mess-Strahlung (14) zu empfangen, **dadurch gekennzeichnet, dass** die Empfangseinrichtung Mess-Strahlung (14), die von dem zweiten Körperteil (26) reflektiert oder reemittiert wurde, empfängt und aus einer Laufzeit, einer Phasenverschiebung, einer Frequenzverschiebung und/oder einer Interferenz mit der ausgesandten Mess-Strahlung (14) die Messwerte und der wenigstens eine Parameter bestimmt werden.

2. Verfahren nach Anspruch 1, **dadurch gekennzeichnet, dass** der zweite Körperteil (26) nicht direkt an den ersten Körperteil (24) angrenzt.

3. Verfahren nach Anspruch 1 oder 2, **dadurch gekennzeichnet, dass** der wenigstens eine Parameter eine Relativposition, Relativbewegung und/oder Relativgeschwindigkeit und/oder Relativbeschleunigung und/oder ein Relativwinkel ist, jeweils des zweiten Körperteiles (26) relativ zu dem ersten Körperteil (24) und/oder eines ersten Teils des zweiten Körperteiles (26) zu einem zweiten Teil des zweiten Körperteils (26).

4. Verfahren nach einem der vorstehenden Ansprüche, **dadurch gekennzeichnet,**
**dass** der wenigstens eine Sensor (34) eingerichtet ist, einen Absolutwinkel, einen Relativwinkel, eine Geschwindigkeit, eine Beschleunigung, eine Kraft, einen Druck, eine Druckwelle, ein Moment, ein elektrisches Feld und/oder ein magnetisches Feld zu detektieren.

5. Verfahren nach einem der vorstehenden Ansprüche, **dadurch gekennzeichnet,**
**dass** das erste Körperteil (24) eine ipsilaterale Extremität (4) oder ein Teil davon, insbesondere ein Fuß (12) ein Knöchel und/oder ein Knie ist und das zweite Körperte (26) eine andere Extremität, vorzugsweise eine kontralaterale Extremität (2), oder ein Teil davon, bevorzugt ein Fuß (12), ein Knöchel und/oder ein Knie, ist.

6. Verfahren nach einem der vorstehenden Ansprüche, **dadurch gekennzeichnet,**
**dass** der wenigstens eine Sensor (34) an einem Bauteil der orthopädietechnischen Einrichtung oder dem ersten Körperteil (24) angeordnet ist und zumindest auch Messwerte detektiert, aus denen der wenigstens eine Parameter des zweiten Körperteiles (26) bestimmt wird.

7. Verfahren nach einem der vorstehenden Ansprüche, **dadurch gekennzeichnet, dass** die Mess-Strahlung (14) Ultraschall-Wellen und/oder eine elektromagnetische Mess-Strahlung (14), besonders bevorzugt Radarstrahlung und/oder sichtbares Licht und/oder IR-Strahlung ist.

8. Verfahren nach einem der vorstehenden Ansprüche, **dadurch gekennzeichnet,**
**dass** an dem zweiten Körperteil (26) wenigstens ein Transponder und/oder ein Tag und/oder ein Reflektor für die ausgesandte Mess-Strahlung (14) angeordnet ist.

9. Verfahren nach einem der vorstehenden Ansprüche, **dadurch gekennzeichnet, dass** zum Bestimmen des wenigstens einen Parameters, insbesondere zum Bestimmen des wenigstens einen Parameters des zweiten Körperteils (26), Daten der orthopädietechnischen Einrichtung und/oder des Trägers verwendet werden.

10. Verfahren nach einem der vorstehenden Ansprüche, **dadurch gekennzeichnet, dass** wenigstens eine Steuergröße des wenigstens einen steuerbaren Aktuators auf einen Sollwert oder einen Sollwertverlauf gesteuert wird, der abhängig ist von dem erkannten Bewegungszustand (30) und von dem wenigstens einen Parameter des funktionalen Körperteils, bevorzugt der kontralateralen Extremität (2).

11. Orthopädietechnische Einrichtung zum Anordnen an einer ipsilateralen Extremität (4), wobei die orthopädietechnische Einrichtung wenigstens einen Sensor (34) und eine elektrische Steuerung aufweist, die eingerichtet ist zum Durchführen eines Verfahrens nach einem der vorstehenden Ansprüche.

## Claims

1. A method for operating an orthopedic device which
- supports or replaces a first body part (24) of a wearer, and
- comprises at least one controllable actuator,
wherein the method comprises the following step:
a) determining a chronological profile (28) of at least one parameter, which allows for a conclusion to be drawn about a movement status (30) of the wearer, from measurement values of at least one sensor (34),
b) detecting the movement status (30) from the at least one determined chronological profile (28), and
c) controlling the at least one controllable actuator depending on the detected movement status (30),
wherein at least also the chronological profile (28) of at least one parameter of a second body part (26) of the wearer is used to detect the movement status, wherein the at least one sensor (34) comprises at least one transmission device and at least one reception device, wherein the transmission device emits measuring radiation (14) and the reception device is configured to receive measuring radiation (14),
**characterized in that** the reception device receives measuring radiation (14) reflected or re-emitted by the second body part (26), and the measurement values and the at least one parameter are determined from a transit time, a phase shift, a frequency shift and/or interference with the emitted measuring radiation (14).

2. The method according to claim 1, **characterized in that** the second body part (26) does not directly abut the first body part (24).

3. The method according to claim 1 or 2, **characterized in that** the at least one parameter is a relative position, relative movement and/or relative speed and/or relative acceleration and/or relative angle of the second body part (26) to the first body part (14) and/or of a first part of the second body part (26) to a second part of the second body part (26).

4. The method according to one of the preceding claims, **characterized in that** the at least one sensor (34) is configured to detect an absolute angle, a relative angle, a speed, an acceleration, a force, a pressure, a pressure wave, a moment, an electrical field and/or a magnetic field.

5. The method according to one of the preceding claims, **characterized in that** the first body part (24) is an ipsilateral limb (4) or a part thereof, particularly a foot (12), an ankle and/or a knee, and the second body part (26) another limb, preferably a contralateral limb (2) or a part thereof, preferably a foot (12), an ankle and/or a knee.

6. The method according to one of the preceding claims, **characterized in that** the at least one sensor (34) is arranged on a component of the orthopedic device or on the first body part (24) and at least also detects measurement values from which the at least one parameter of the second body part (26) is determined.

7. The method according to one of the preceding claims, **characterized in that** the measuring radiation is ultrasonic waves and/or electromagnetic measuring radiation, especially preferably radar radiation and/or visible light and/or infrared radiation.

8. The method according to one of the preceding claims, **characterized in that** at least one transponder and/or a tag and/or a reflector for the emitted measuring radiation (14) is arranged on the second body part (26).

9. The method according to one of the preceding claims, **characterized in that** data of the orthopedic device and/or the wearer is used to determine the at least one parameter, especially to determine the at least one parameter of the second body part (26).

10. The method according to one of the preceding claims, **characterized in that** at least one control variable of the at least one controllable actuator is controlled to a set point or a set point profile, which is dependent on the detected movement status (30) and the at least one parameter of the functional body part, preferably the contralateral limb (2).

11. An orthopedic device for arranging on an ipsilateral limb (4), the orthopedic device comprising at least one sensor (34) and one electric control unit that is configured to conduct a method according to one of the preceding claims.

## Revendications

1. Procédé pour faire fonctionner un dispositif orthopédique qui
- soutient ou remplace une première partie du corps (24) d'un porteur et qui
- comporte au moins un actionneur commandable,
le procédé comprenant les étapes suivantes consistant à :
a) déterminer une courbe temporelle (28) d'au moins un paramètre permettant de donner une information sur un état de mouvement (30) du porteur, à partir des valeurs de mesure d'au moins un capteur (34),
b) détecter l'état de mouvement (30) à partir de ladite au moins une courbe temporelle (28) déterminée, et
c) commander ledit au moins un actionneur commandable en fonction de l'état de mouvement (30) détecté,
dans lequel, pour détecter l'état de mouvement (30), on utilise au moins également la courbe temporelle (28) d'au moins un paramètre d'une deuxième partie du corps (26) du porteur, ledit au moins un capteur (34) comportant au moins un dispositif d'émission et au moins un dispositif de réception, le dispositif d'émission émettant un rayonnement de mesure (14) et le dispositif de réception étant agencé pour recevoir le rayonnement de mesure (14),
**caractérisé en ce que** le dispositif de réception reçoit le rayonnement de mesure (14) qui a été réfléchi ou réémis par la deuxième partie du corps (26), et les valeurs de mesure et ledit au moins un paramètre sont déterminés à partir d'un temps de transit, d'un déphasage, d'un décalage de fréquence et/ou d'une interférence avec le rayonnement de mesure (14) émis.

2. Procédé selon la revendication 1,
**caractérisé en ce que** la deuxième partie du corps (26) n'est pas directement adjacente à la première partie du corps (24).

3. Procédé selon la revendication 1 ou 2,
**caractérisé en ce que** ledit au moins un paramètre est une position relative, un mouvement relatif et/ou une vitesse relative et/ou une accélération relative et/ou un angle relatif, respectivement de la deuxième partie du corps (26) par rapport à la première partie du corps (24) et/ou d'un premier élément de la deuxième partie du corps (26) par rapport à un deuxième élément de la deuxième partie du corps (26).

4. Procédé selon l'une des revendications précédentes,
**caractérisé en ce que** ledit au moins un capteur (34) est agencé pour détecter un angle absolu, un angle relatif, une vitesse, une accélération, une force, une pression, une onde de pression, un moment, un champ électrique et/ou un champ magnétique.

5. Procédé selon l'une des revendications précédentes,
**caractérisé en ce que** la première partie du corps (24) est un membre ipsilatéral (4) ou une partie de celui-ci, en particulier un pied (12), une cheville et/ou un genou, et la deuxième partie du corps (26) est un autre membre, de préférence un membre controlatéral (2), ou une partie de celui-ci, de préférence un pied (12), une cheville et/ou un genou.

6. Procédé selon l'une des revendications précédentes,
**caractérisé en ce que** ledit au moins un capteur (34) est disposé sur un composant du dispositif orthopédique ou sur la première partie du corps (24) et détecte au moins également des valeurs de mesure à partir desquelles est déterminé ledit au moins un paramètre de la deuxième partie du corps (26).

7. Procédé selon l'une des revendications précédentes,
**caractérisé en ce que** le rayonnement de mesure (14) est constitué d'ondes ultrasonores et/ou d'un rayonnement de mesure électromagnétique (14), de préférence d'un rayonnement radar et/ou d'une lumière visible et/ou d'un rayonnement infrarouge.

8. Procédé selon l'une des revendications précédentes,
**caractérisé en ce qu'**au moins un transpondeur et/ou une étiquette et/ou un réflecteur pour le rayonnement de mesure émis (14) est disposé sur la deuxième partie du corps (26).

9. Procédé selon l'une des revendications précédentes,
**caractérisé en ce que**, pour déterminer ledit au moins un paramètre, en particulier pour déterminer ledit au moins un paramètre de la deuxième partie du corps (26), on utilise des données provenant du dispositif orthopédique et/ou du porteur.

10. Procédé selon l'une des revendications précédentes,
**caractérisé en ce qu'**au moins une grandeur de commande dudit au moins un actionneur commandable est régulée à une valeur de consigne ou à une courbe de valeur de consigne qui dépend de l'état de mouvement détecté (30) et dudit au moins un paramètre de la partie fonctionnelle du corps, de préférence du membre controlatéral (2).

11. Dispositif orthopédique destiné à être placé sur un membre ipsilatéral (4), le dispositif orthopédique comportant au moins un capteur (34) et une commande électrique qui est agencée pour mettre en œuvre un procédé selon l'une des revendications précédentes.
